# EUROPEAN PATENT APPLICATION

(11) **EP 2 518 502 A1**
(43) Date of publication of application: **31.10.2012**
(21) Application number: 10838966.9
(22) Date of filing: 22.12.2010
(51) Int. Cl.: G01N 33/543, G01N 21/17, G01N 33/49

(54) **METHOD FOR DETERMINING HEMAGGLUTINATION IMAGE AND DEVICE FOR DETERMINING HEMAGGLUTINATION IMAGE**

(30) Priority: 24.12.2009 JP 2009293376
(71) Applicant: Beckman Coulter, Inc., Brea, CA 92821 (US)
(72) Inventor: HAGA, Tadashi, Sunto-gun Shizuoka 411-0931 (JP)
(74) Representative: Müller-Boré & Partner Patentanwälte
(86) International application number: PCT/JP2010/007451
(87) International publication number: WO 2011/077728

(57) **Abstract**

The present invention provides a blood cell agglutination image determining method and a blood cell agglutination image determining apparatus capable of processing a blood sample in a short time and obtaining a reproducible determination result. Provided are a blood cell agglutination image determining method and a blood cell agglutination image determining apparatus for determining a blood sample to be positive or negative based on a blood cell agglutination image of a reaction between a blood sample and a reagent in a reaction container. The apparatus comprises: a rotation mechanism **R** for rotating a reaction container so that a bottom wall of the reaction container will turn outwards by centrifugal force; and an inclining apparatus **7** for inclining the reaction container so that a front part of the reaction container along the rotating direction will be downwards with respect to the vertical direction more than a back part thereof.

## Description

### TECHNICAL FIELD

The present invention relates to a blood cell agglutination image determining method and a blood cell agglutination image determining apparatus for determining a blood type, an infectious disease, or the like in the medical field.

### BACKGROUND ART

Tube tests, column agglutination technology, or plate settling methods in general have been conventionally known as a method for examining blood, e.g., a method for determining a blood type, on the basis of a reaction image from an antigen-antibody reaction.

In the tube tests, 50 µL of blood cells as a reagent and 50 µL of blood plasma as a sample are dispensed into a tube having an internal diameter of 10 mm, and are processed by centrifugation (900 G to 1,000 G, 15 seconds), so that blood cell pellets formed by the agglutination reaction between the blood cells and the blood plasma will be precipitated at the bottom of the tube. Subsequently, a blood type is determined from a result of agglutination (positive) or non-agglutination (negative), which is determined by visual observation on the basis of either blood cell agglutinates or un-agglutinated blood cells that are the blood cell pellets precipitated at the bottom of the tube, but re-suspending as blood cell agglutinates or un-agglutinated blood cells by the agitating of the tube (see, for example, Non-Patent Literature 1).

In the column agglutination technology, a blood type is determined, using a column in which inactive particles and an aqueous medium are filled, from a result of agglutination (positive) or non-agglutination (negative), which is determined by optically visualizing a complex obtained by the agglutination reaction between a carrier binding antibody and an antigen in the aqueous medium, or a complex obtained by the agglutination reaction between a carrier binding antigen and an antibody, when the blood of an examination subject is dispensed into the column (see, for example, Patent Literature 1).

In the plate settling method, using a microplate having a plurality of wells arranged in a matrix, a blood type is determined from a result of agglutination (positive) or non-agglutination (negative), which is determined on the basis of a blood cell agglutination image of a blood sample and a reagent dispensed into each of the wells (see, for example, Patent Literature 2).

### [Citation List]

### [Patent Literature]

Patent Literature 1: Japanese Patent No. 3299768
Patent Literature 2: Japanese Publication for Opposition No. 63-60854

### [Non-Patent Literature]

Non-Patent Literature 1: "Yuketsu Kensa No Jissai", Library XII of Japanese Association of Medical Technologists, Revised Version, 3rd Edition, P. 16

### SUMMARY OF THE INVENTION

### [Technical Problem]

The present invention provides a blood cell agglutination image determining method comprising: a reaction step of allowing a blood sample to react with a reagent in a reaction container; a centrifugation processing step of rotating the reaction container so that a bottom wall of the reaction container will turn outwards by centrifugal force; an inclining step of inclining the reaction container so that a front part of the reaction container along the rotating direction in the centrifugation processing step will be downwards with respect to the vertical direction more than a back part of the reaction container; and a determining step of determining the blood sample to be positive or negative on the basis of a blood cell agglutination image from a reaction formed in the reaction container between the blood sample and the reagent.

In another aspect, the blood cell agglutination image determining method according to the present invention is a blood cell agglutination image determining method for determining a blood sample to be either positive or negative on the basis of a blood cell agglutination image of a reaction between the blood sample and a reagent in a reaction container, characterized by comprising: a centrifugation processing step of rotating the reaction container so that a bottom wall of the reaction container will turn outwards by centrifugal force; and an inclining step of inclining the reaction container so that a front part of the reaction container along the rotating direction in the centrifugation processing step will be downwards with respect to the vertical direction more than a back part of the reaction container.

In one embodiment, with regard to the blood cell agglutination image determining method according to the present invention, in the invention above, the reaction container is a well formed in a microplate.

In one embodiment, with regard to the blood cell agglutination image determining method according to the present invention, in the invention above, a tiered portion is formed on an inner surface of a bottom wall of the reaction container or well, and the blood cell is a red blood cell.

In one embodiment, with regard to the blood cell agglutination image determining method according to the present invention, in the invention above, after the reaction or inclining step, the method further comprises: a canceling step of canceling the inclination of the reaction container; an image-capturing step of capturing an image of the reaction container, inclination of which is canceled; an image processing step of processing an image of the reaction container including a reaction image captured in the image-capturing step, and calculating a determination value on the basis of the image of the reaction container; and a determining step of determining the blood sample to be positive or negative on the basis of the determination value calculated in the image processing step. Alternatively, with regard to the blood cell agglutination image determining method according to the present invention comprising the determination step, in the invention above, after the inclining step, the method further comprises: a canceling step of canceling the inclination of the reaction container; an image-capturing step of capturing an image of the reaction container, inclination of which is canceled; and an image processing step of processing an image of the reaction container including a reaction image captured in the image-capturing step, and calculating a determination value on the basis of the image of the reaction container, wherein the determining step comprises a determining step of determining the blood sample to be positive or negative on the basis of the determination value calculated in the image processing step.

In various embodiments, the method according to the present invention comprises any two or more of the characteristics described above.

In another aspect, the present invention provides a blood cell agglutination image determining apparatus comprising: a reaction container housing section for housing a reaction container for allowing a blood sample to react with a reagent therein; rotating means for rotating the reaction container so that a bottom wall of the reaction container will turn outwards by centrifugal force; inclining means for inclining the reaction container so that a front part of the reaction container along the rotating direction will be downwards with respect to the vertical direction more than a back part of the reaction container; and determining means for determining the blood sample to be positive or negative on the basis of a blood cell agglutination image from a reaction formed in the reaction container between the blood sample and the reagent.

Further, the blood cell agglutination image determining apparatus according to the present invention is a blood cell agglutination image determining apparatus for determining a blood sample to be either positive or negative on the basis of a blood cell agglutination image of a reaction between the blood sample and a reagent in a reaction container, the apparatus comprising: rotatingmeans for rotating the reaction container so that a bottom wall of the reaction container will turn outwards by centrifugal force; and inclining means for inclining the reaction container so that a front part of the reaction container along the rotating direction will be downwards with respect to the vertical direction more than a back part of the reaction container.

In one embodiment, with regard to the blood cell agglutination image determining apparatus according to the present invention, in the invention above, the reaction container is a well formed in a microplate.

In one embodiment, with regard to the blood cell agglutination image determining apparatus according to the present invention, in the invention above, a tiered portion is formed on an inner surface of a bottom wall of the reaction container or well, and the blood cell is a red blood cell.

In one embodiment, with regard to the blood cell agglutination image determining apparatus according to the present invention, in the invention above, the rotating means comprises: a motor; and a rotor rotated by the motor, and the reaction container housing section comprises a bucket for retaining the reaction container or a microplate, the bucket being swingably supported by the rotor around the horizontal axis.

In one embodiment, with regard to the blood cell agglutination image determining apparatus according to the present invention comprising determining means, in the invention above, the apparatus further comprises: image-capturing means for capturing an image of the reaction container; and image processing means for processing an image of the reaction container including a reaction image captured by the image-capturing means,and calculating a determination value on the basis of the image of the reaction container, wherein the determining means comprises determining means for determining the blood sample to be positive or negative on the basis of the determination value.

In one embodiment, with regard to the blood cell agglutination image determining apparatus according to the present invention comprising determining means, in the invention above, the apparatus further comprises: image-capturing means for capturing an image of a plurality of the wells; and image processing means for processing an image of each of the wells including a reaction image captured by theimage-capturing means,and calculating a determination value on the basis of the image of each of the wells, wherein the determining means comprises determining means for determining the blood sample to be positive or negative for each of the wells on the basis of the determination value.

In one embodiment, with regard to the blood cell agglutination image determining apparatus according to the present invention, in the invention above, the apparatus comprises: image-capturing means for capturing an image of a plurality of the wells; image processing means for processing an image of each of the wells including a reaction image captured by the image-capturing means, and calculating a determination value on the basis of the image of each of the wells; and determining means for determining the blood sample to be positive or negative on the basis of the determination value.

In one embodiment, with regard to the blood cell agglutination image determining apparatus according to the present invention, in the invention above, the inclining means comprises: holding means for holding an outer edge of the reaction container housing section; revolving means for revolving the holding means to incline the reaction container housing section so that a shorter side of the reaction container in the front of the rotating direction will be downwards with respect to the vertical direction; and elevating means for elevating the revolving means together with the holding means.

In one embodiment, with regard to the blood cell agglutination image determining apparatus according to the present invention, in the invention above, the rotating means comprises: a motor; and a rotor rotated by the motor, wherein the reaction container housing section comprises a bucket for retaining the microplate, the bucket being swingably supported by the rotor around the horizontal axis, and wherein the inclining means comprises: holding means for holding an outer edge of the bucket; revolving means for revolving the holding means to incline the bucket so that a shorter side of the microplate in the front of the rotating direction will be downwards with respect to the vertical direction; and elevating means for elevating the revolving means together with the holding means.

In various embodiments, the apparatus according to the present invention comprises any one or a plurality of (two or more) of the characteristics described above of the blood cell agglutination image determining apparatus and method according to the present invention.

In another aspect, the present invention provides a control program for sample processing in a blood cell agglutination image determining apparatus for determining a blood sample to be positive or negative on the basis of a blood cell agglutination image of a reaction between the blood sample and a reagent in a reaction container, the control program for implementing processing that is executed by the blood cell agglutination image determining apparatus in accordance with an instruction from an operator, the processing comprising: a centrifugation processing procedure for rotating the reaction container so that a bottom wall of the reaction container will turn outwards by centrifugal force; and an inclining procedure for inclining the reaction container so that a front part of the reaction container along the rotating direction in the centrifugation processing step will be downwards with respect to the vertical direction more than a back part of the reaction container.

In various embodiments, the program according to the present invention comprises any one or a plurality of the characteristics described above of the apparatus and method according to the present invention.

In another aspect, the present invention provides a computer-readable recording medium recording a control program for sample processing in a blood cell agglutination image determining apparatus for determining a blood sample to be positive or negative on the basis of a blood cell agglutination image of a reaction between the blood sample and a reagent in a reaction container, the control program for implementing processing that is executed by the blood cell agglutination image determining apparatus in accordance with an instruction from an operator, the processing comprising: a centrifugation processing procedure for rotating the reaction container so that a bottom wall of the reaction container will turn outwards by centrifugal force; and an inclining procedure for inclining the reaction container so that a front part of the reaction container along the rotating direction in the centrifugation processing step will be downwards with respect to the vertical direction more than a back part of the reaction container.

In various embodiments, the recording medium according to the present invention comprises any one or a plurality of the characteristics described above of the apparatus and method according to the present invention.

### [Advantageous Effects of Invention]

According to the present invention, a reaction container is rotated so that a bottom wall of the reaction container will turn outwards by centrifugal force, and the reaction container is inclined so that a front part of the reaction container along the rotating direction will be downwards with respect to the vertical direction more than a back part of the reaction container. Thereby the present invention exerts an effect of providing a blood cell agglutination image determining method and a blood cell agglutination image determining apparatus capable of processing a blood sample in a shorter time and obtaining a reproducible determination result.

### BRIEF DESCRIPTION OF THE DRAWINGS

Figure 1 is a schematic configuration diagram of a blood cell agglutination image determining apparatus according to an embodiment of the present invention.
Figure 2 is a perspective view of a rotor.
Figure 3 is a perspective view of a rotor having buckets attached thereto.
Figure 4 is a plane view of a rotor having buckets attached thereto, where a microplate is set with each bucket.
Figure 5 is a plane view of a microplate.
Figure 6 is a cross sectional view along the line C-C in Figure 5.
Figure 7 is a perspective view illustrating an enlarged cross section of a well formed in a microplate.
Figure 8 is a flowchart for describing a blood cell agglutination image determining method according to an embodiment of the present invention.
Figure 9 is a front view for schematically describing the state of a bucket in relation to the rotation of a rotor in a blood cell agglutination image determining apparatus.
Figure 10 is a front view schematically illustrating a state where a bucket is detached from a rotor by an inclining apparatus.
Figure **11** is a front view schematically illustrating a state where a bucket is inclined by an inclining apparatus.
Figure **12** is a diagram illustrating a reaction image of a well when blood plasma (blood serum) processed using a plate settling method is negative.
Figure **13** is a diagram illustrating a reaction image of a well when blood plasma (blood serum) processed using a plate settling method is weak positive.
Figure **14** is a diagram illustrating a reaction image of a well when blood plasma (blood serum) processed using a plate settling method is strongly positive.
Figure **15** is a diagram illustrating an embodiment of the present invention where an image of a microplate immediately after centrifugation processing is performed by the blood cell agglutination image determining apparatus is captured by a CCD camera.
Figure **16** is a diagram illustrating an embodiment of the present invention where an image of a microplate after centrifugation processing and further inclination for two minutes is captured by a CCD camera.
Figure **17** is a diagram illustrating an embodiment of the present invention where determination results of the images illustrated in Figure **16** are described together with the concentration of red blood cell suspensions.
Figure **18** is an enlarged view of a strongly positive well in the fifth row, second column among the images illustrated in Figure **17****.**
Figure **19** is an enlarged view of a weak positive well in the fifth row, third column among the images illustrated in Figure **17****.**
Figure **20** is an enlarged view of a negative well in the fifth row, fifth column among the images illustrated in Figure **17****.**
Figure **21** is a diagram illustrating a comparative example of the present invention where an image of a microplate immediately after centrifugation processing is performed is captured by a CCD camera.
Figure **22** is a diagram illustrating a comparative example of the present invention where an image of a microplate after being inclined for two minutes by an inclining apparatus is captured by a CCD camera.
Figure **23** is an enlarged view of a well in the fourth row, first column.
Figure **24** is an enlarged view of a well in the fourth row, second column.
Figure **25** is an enlarged view of a well in the fourth row, fourth column.

### EMBODIMENTS FOR CARRYING OUT THE INVENTION

Hereinafter, embodiments of a blood cell agglutination image determining method and a blood cell agglutination image determining apparatus according to the present invention will be described in detail with reference to the accompanying drawings. It should be noted that the present invention will not be limited by the present examples.

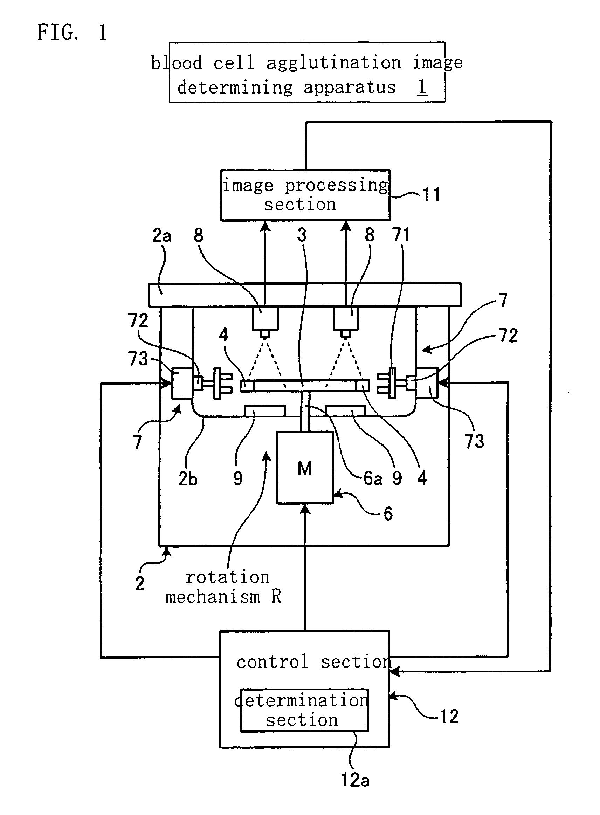

Figure **1** is a schematic configuration diagram of a blood cell agglutination image determining apparatus according to an embodiment of the present invention. As illustrated in Figure **1****,** a blood cell agglutination image determining apparatus **1** comprises a casing **2;** a rotor **3;** a motor **6;** an inclining apparatus **7;** a CCD camera **8;** an image processing section **11;** and a control section **12.**

The casing **2** includes a door **2a** as a lid for capping in a freely openable and closable manner in the upward direction, and the rotor **3** is positioned within a chamber **2b.**

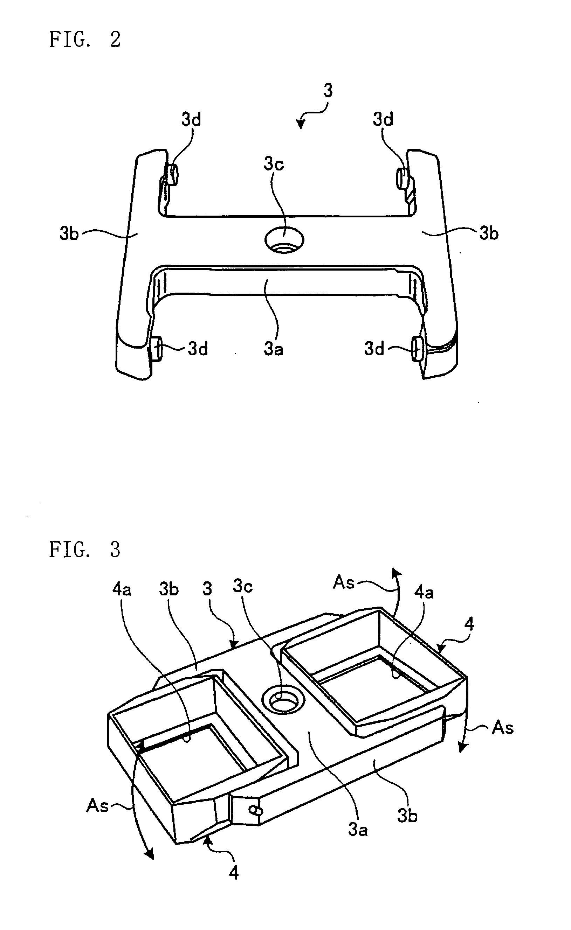

Figure **2** is a perspective view of the rotor **3.** As illustrated in Figure **2****,** the rotor **3** is an H-shaped member having yokes **3b** on both sides of a main body **3a,** and an insertion hole **3c** is formed in the center of the main body **3a.** Each yoke **3b** includes trunnion pins **3d** provided therefor at internally opposing positions, where each of the trunnion pins **3d** protrudes internally and is a horizontal shaft for horizontally supporting a bucket **4.** The rotor **3** balances and retains buckets **4** on both sides of the main body **3a** by means of the trunnion pins **3d** illustrated in Figure **2****.** The bucket **4** functions as a reaction container housing section for housing a reaction container, such as a microplate **5.**

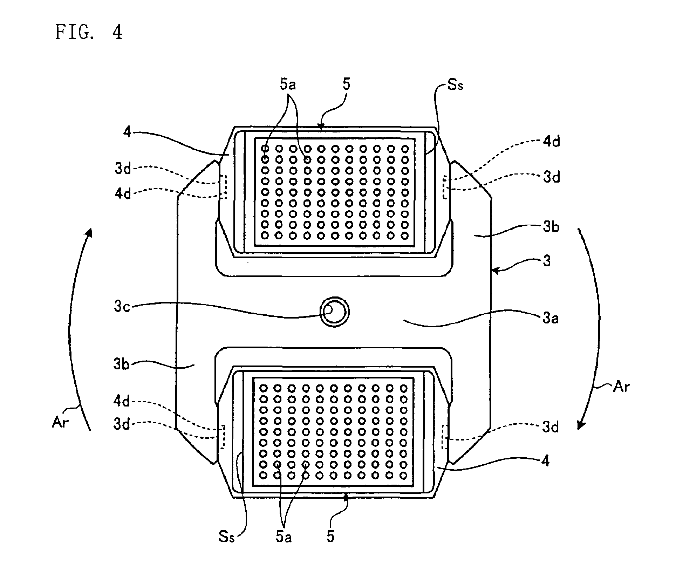

Figure **3** is a perspective view of a rotor **3** having buckets **4** attached thereto. Figure **4** is a plane view of a rotor **3** having buckets **4** attached thereto, where a microplate **5** is set with each bucket **4.** As illustrated in Figure **3****,** the bucket **4** is a rectangular parallelepiped casing with an opened upper part and with an opening **4a** formed on a bottom surface thereof. As illustrated in Figure **4****,** the bucket **4** includes an engagement groove **4b** formed at a position slightly displaced towards the outer circumference of the rotor **3** from the center, and between the trunnion pins **3d** on both sides. Each trunnion pin **3d** is engaged with the engagement groove **4b** so that the bucket **4** will be supported by the rotor **3** as illustrated in Figure **3****,** and the bucket **4** swings as illustrated by the arrow **As** with trunnion pins (not shown) as the center. Additionally, as illustrated in Figure **4****,** the bucket **4** retains the microplate **5** and rotates in the direction illustrated by the arrow **Ar** together with the rotor **3 ,** with the insertionhole **3c** as the center. During the rotation, the engagement groove **4b** of the bucket **4** is slightly displaced towards the outer circumference of the rotor **3.** Thus, as the rotor **3** rotates, the side of the bucket **4** closer to the internal circumference of the rotor **3** swings downwards together with the microplate **5,** with the trunnion pins **3d** as the center.

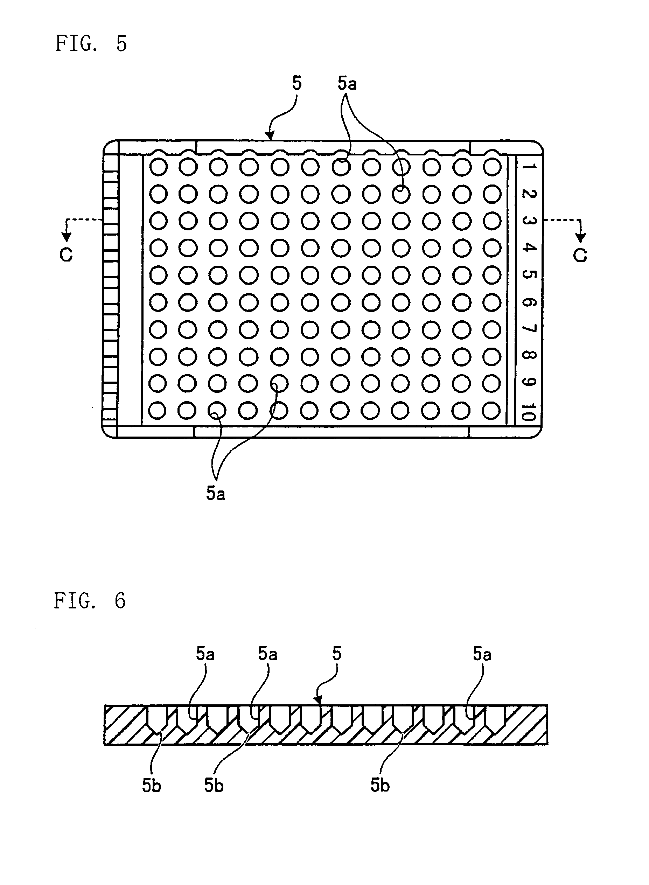

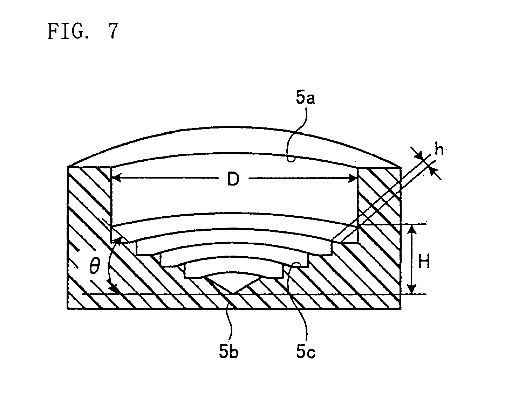

Figure **5** is a plane view of a microplate. Figure **6** is a cross sectional view along the line C-C in Figure **5****.** Further, Figure **7** is a perspective view illustrating an enlarged cross section of a well formed in a microplate. Hereinafter, the structure of the microplate will be described with reference to Figures **5** to **7****.**

As illustrated in Figure **5****,** the microplate **5** comprises 12 x 10 wells **5a** formed and arranged therein in a matrix. A well **5a** is a very small reaction container, in which a dispensed blood sample and a dispensed reagent react with each other, and as illustrated in Figure **6****,** the well **5a** has abottomwall **5b,** the inner surface of which has a substantially conical shape. Here, with regard to the well **5a** illustrated in Figure **7****,** when the diameter on the upper surface of the microplate **5** is defined to be **D** (= 6 mm) and the depth of the inner surface portion of the bottom wall **5b** is defined to be **H,** the average angle of inclination *θ* of the inner surface [= tan⁻¹ (H/(D/2))] is set to be 30 degrees. Additionally, a tiered portion **5c** is formed on the inner surface of the bottom wall **5b,** the tiered portion consisting of a plurality of step portions formed in concentric circles and the tiered portion having a height **h,** where **h** = 16 µm.

As illustrated in Figure **1****,** together with the rotor **3** and the bucket **4 ,** the motor **6** constitutes a rotationmechanism **R** for rotating the microplate **5,** and the motor **6** rotates the rotor **3** to move blood cells contained in the blood sample or reagent dispensed in each well **5a** towards the bottom wall **5b.** The motor **6** comprises the rotor **3** attached thereto by a nut (not shown) screwed into an upper end of a rotation shaft **6a** inserted into the insertion hole **3c.**

The inclining apparatus **7** is provided for each bucket **4,** and is means for inclining the microplate **5,** with a shorter side **Ss** (see Figure **4****),** positioned in the front of the bucket **4** along the rotating direction, to be downwards with respect to the vertical direction. As illustrated in Figure **1****,** the inclining apparatus **7** is provided at a position facing the bucket **4** at the side surface of the chamber **2b,** and the inclining apparatus **7** comprises a chuck **71,** a revolving motor **72** and an elevating device **73.**

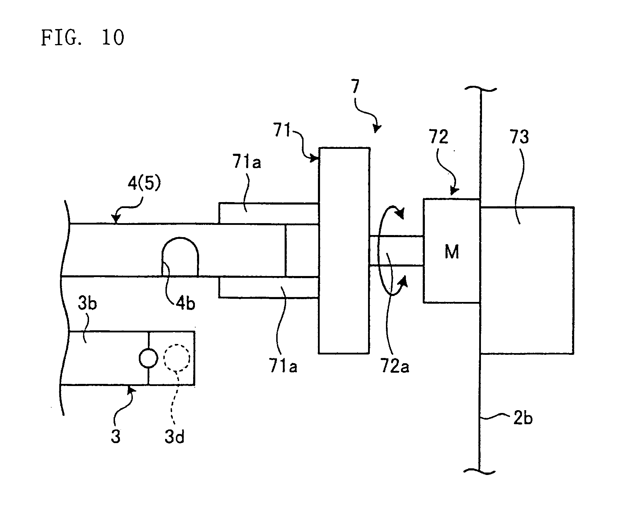

As illustrated in Figure **1****,** the chuck **71** holds an outer edge of the longer side of the bucket **4** by two holding pawls **71a** (see Figure **10****) .** The revolving motor **72** comprises a revolving shaft **72a** (see Figure **10****),** and revolves the chuck **71** provided at the end portion of the revolving shaft **72a** to incline the bucket **4** so that the shorter side **Ss** (see Figure **4****)** of the microplate **5** in the front of the rotating direction will be downwards with respect to the vertical direction. The elevating device **73** elevates the rotation motor **72** using a ball screw, and the rotation motor **72** is attached to a slider (not shown).

A CCD camera **8** is means for capturing images of a plurality of wells **5a** of a microplate **5,** and the CCD camera **8** outputs image signals of a plurality of captured wells **5a** to an image processing section **11.** As illustrated in Figure **1****,** the CCD camera **8** is provided at a position facing the microplate **5** that is rotated by the rotor **3** below the door **2a.** Here, the inclining apparatus **7** and the CCD camera **8** are arranged at positions apart from each other at the central angle of 90 degrees along the rotational locus of the microplate **5.**

A lighting **9** is lighting means, such as a fluorescent lamp, provided for a lower part of the inner wall of the chamber **2b,** for lighting the microplate **5** from below.

The image processing section **11** performs image processing on an image of each well **5a** on the basis of the image signals of the plurality of wells **5a** that are inputted from the CCD camera **8,** calculates a determination value for each well **5a,** and outputs the thus obtained determination value for each well **5a** to a determination section **12a.**

The control section **12** controls respective sections by providing instructions of operation timing, transferring data, or the like to the respective sections that constitute the blood cell agglutination image determining apparatus **1,** to control the operation of the overall blood cell agglutination image determining apparatus **1** collectively. The control section **12** constitutes a microcomputer or the like with a built-in memory for retaining various data necessary for the operation of the blood cell agglutination image determining apparatus **1,** in addition to determination results regarding the blood type of each blood sample on the basis of the determination result of each well **5a** or determination results of a plurality of wells **5a;** and as illustrated in Figure **1****,** the control section **12** comprises the determination section **12a.** The determination section **12a** determines a blood sample with three stages of strongly positive, weak positive or negative for each well **5a,** wherein strongly positive is where blood cell agglutination has occurred the most, on the basis of the determination value for each well **5a** input from the image processing section **11.**

For example, in determining a blood type, the blood cell agglutination image determining apparatus 1 constituted as described above dispenses undiluted blood plasma, or blood plasma diluted down to a predetermined concentration with physiological saline, and determines the blood sample to be either positive or negative for each well **5a** of the microplate **5** on the basis of an agglutination image resulted from the reaction between the blood plasma and blood cells when the blood cells were dispensed at a predetermined ratio. Here, a reaction step of allowing a blood sample and a reagent to react with each other in a reaction container may be in progress at any time in relation to a centrifugation processing step and an inclination step. The reaction step may be in progress prior to either or both of those steps, or the reaction step may be in progress in parallel to, or after, those steps.

Here, if the blood plasma and blood cells react with each other to form an agglutination image, then the sample is positive; and if an agglutination image fails to form, then the sample is negative. Additionally, for the case of ABO typing for examining the blood type of red blood cells, red blood cells taken from blood are used as a blood sample, and an anti-A antibody and an anti-B antibody for determining the blood type are used as reagents. Further, for the case of ABO reverse testing for examining the blood type of blood plasma (blood serum), the blood plasma (blood serum) is used as a blood sample, and a type-A red blood cell suspension and a type-B red blood cell suspension are used as reagents, where type-A red blood cells of blood of a known blood type are suspended in physiological saline, and type-B red blood cells of blood of a known blood type are suspended in physiological saline. Hereinafter, an explanation will be provided with a case of the ABO reverse testing for examining the blood type of blood plasma (blood serum).

First, the blood plasma (blood serum) of an object for determination is dispensed to two wells **5a,** then the type-A red blood cell suspension and the type-B red blood cell suspension are dispensed in these two wells **5a,** and the microplate **5** after the dispensing is attached to a bucket **4.** Then, the door **2a** of the blood cell agglutination image determining apparatus **1** is opened upwards, and two of such buckets **4** each having a microplate **5** attached thereto are retained by the rotor **3** within the chamber **2b.**

Here, the engagement groove **4b** (see Figure **10****)** of the bucket **4** is engaged with the trunnion pins **3d,** so that the bucket **4** is supported by the trunnionpins **3d**as illustrated in Figure **4****.** Thus, the rotor **3** retains two microplates **5** in a point symmetry manner in relation to the insertion hole **3c** of the rotor **3,** which is the center of rotation.

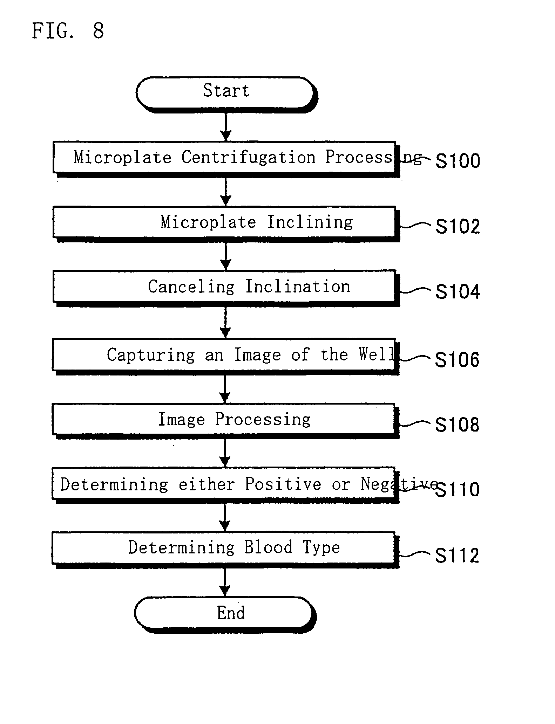

Next, the operator closes the door **2a** of the blood cell agglutination image determining apparatus **1,** turns on the switch of the blood cell agglutination image determining apparatus **1,** and presses a blood cell agglutination process determining button. Then, in accordance with a pre-set program, the blood cell agglutination image determining apparatus **1** drives the motor **6** to rotate the rotor **3,** thus performing centrifugation processing on the microplates **5.** Hereinafter, a blood cell agglutination image determining method according to the present invention will be described in detail on the basis of the flowchart illustrated in Figure **8****.**

First, under the control of the control section 12, the blood cell agglutination image determining apparatus 1 rotates the rotor **3** to perform centrifugation processing on the microplates **5** (step **S100).** While blood cells are separated out towards the side closer to the bottom wall **5b** by the centrifugation processing, the control section **12** controls the rotation of the microplates **5** by the motor **6** so that the blood cells will not be completely pressed against the bottom wall **5b.** For such controlling, the control section **12** controlled the motor **6** so that the rotor **3** rotated at a rotation rate of 1,000 rotations per minute (200G) for 15 seconds. In each well **5a,** an agglutination reaction starts due to the antigen-antibody reaction between antibodies present in the blood plasma (blood serum) and antigen epitope present on the surfaces of the red blood cells. Thus, not only non-agglutinated blood cells, but also an agglutination mass produced due to the agglutination reaction, are included in the blood cells moving towards the side closer to the bottom wall **5b.**

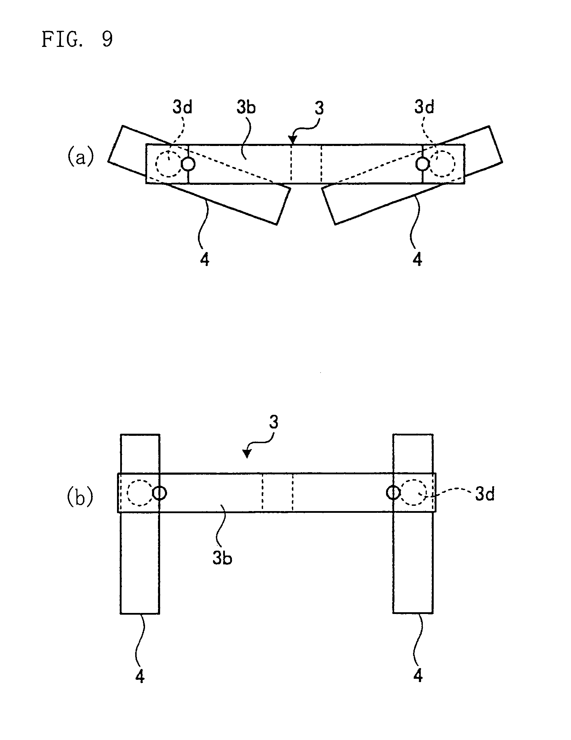

Here, Figure **9** is a front view for describing state of a bucket **4** in relation to the rotation of the rotor **3** in the blood cell agglutination image determining apparatus 1. At the start of the rotation of the motor **6,** the bucket **4** is in such a state where the upper surface of the rotor **3** and the upper surface of the bucket **4** are flush. As the rotation rate of the motor **6** increases, the lower part of the bucket **4** swings outwards from the rotor **3,** due to the working centrifugal force, with the trunnion pins **3d** as the center; and as illustrated in Figure **9 (a)**, the side of the bucket **4** closer to the internal circumference of the rotor **3** moves downwards while the side closer to the outer circumference moves upwards. Then, the rotation rate further increases, and when the rotation rate of the motor **6** reaches 1,000 rotations per minute, the control section **12** stops the motor **6.** Thus, the rotation rate of the bucket **4,** lower part of which is swinging outwards from the rotor **3,** starts to decrease immediately before the bucket **4** becomes orthogonal in relation to the upper surface of the rotor **3** illustrated in Figure **9 (b)** **.** Accordingly, in the microplates **5,** the red blood cells in each well **5a** are moved towards the side closer to the bottom wall **5b.**

Next, the blood cell agglutination image determining apparatus **1** inclines each microplate **5** so that the shorter side **Ss** (see Figure **4****)** of the microplate **5** in the front of the rotating direction will be downwards with respect to the vertical direction (step **S102).**

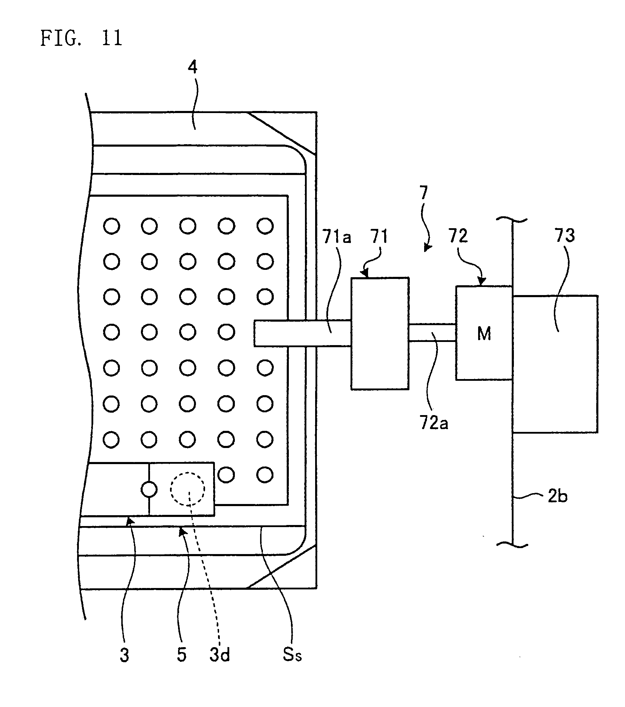

Here, Figure **10** is a front view illustrating a state where the bucket **4** is detached from the rotor **3** by the inclining apparatus **7.** Figure **11** is a front view illustrating a state where the bucket **4** is inclined by the inclining apparatus **7.** Hereinafter, the inclining operation of the microplate **5** by the inclining apparatus **7** will be described with reference to Figures **10** and **11****.**

First, the blood cell agglutination image determining apparatus **1** drives the inclining apparatus **7** so that the chuck **71** holds the outer edge of the longer side of the bucket **4** by two holding pawls **71a.** Thereafter, the revolving motor **72** is raised by the elevating device **73,** and the bucket **4** is detached from the rotor **3** as illustrated in Figure **10****.** Then, the blood cell agglutination image determining apparatus **1** drives the revolving motor **72** to revolve the chuck **71,** and as illustrated in Figure **11****,** retains the bucket **4** to be inclined for two minutes at 90 degrees in relation to the horizontal plane so that the shorter side **Ss** (see Figure **4****)** of the microplate **5** in the front of the rotating direction will be downwards with respect to the vertical direction.

After a lapse of two minutes, the blood cell agglutination image determining apparatus **1** drives the inclining apparatus **7** in the opposite direction from the description above to allow the rotor **3** to retain the bucket **4,** and the incline of the microplate **5** is canceled (step **S104) .** As a result the microplate 5 is retained horizontally with the bucket **4** (see Figure **4****).**

Next, the blood cell agglutination image determining apparatus **1** allows the CCD camera **8** to capture images of a plurality of the wells **5a** of the microplate **5** (step **S106) .** At this stage, since the microplate 5 is lighted by the lighting **9** from below as illustrated in Figure **1****,** the image of the plurality of wells **5a** can be clearly captured. Thereafter, on the basis of image signals of the plurality of wells **5a** input from the CCD camera **8,** the image processing section **11** performs image processing on the image for each well **5a** including a reaction image (step **S108).**

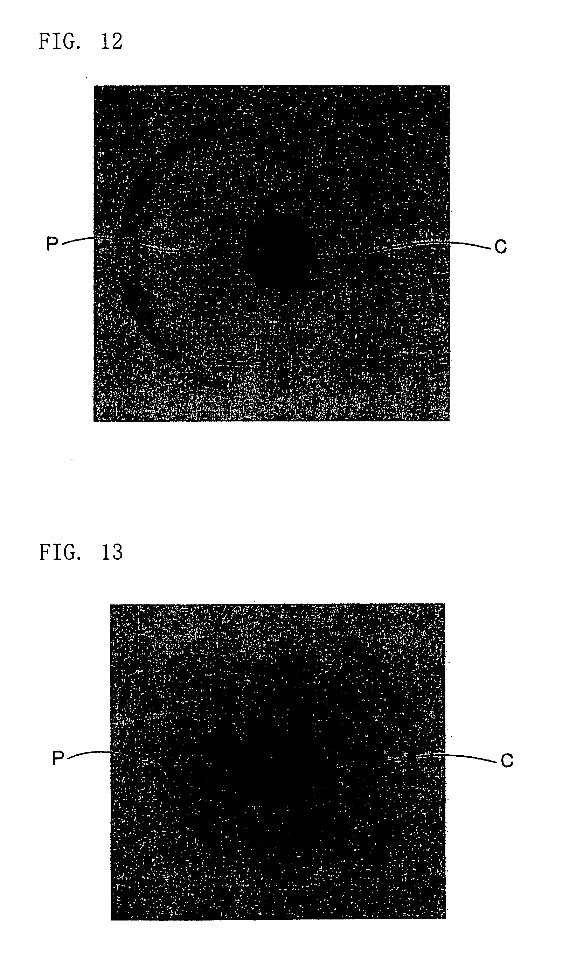

The image processing section **11** performs calculation, for example, with a determination parameter **P/C** of an agglutination reaction, which is obtained by multiplying a value **Lp/Lc** of the ratio between an average amount of light **Lp** in the periphery part and an average amount of light **Lc** in the center part of a well **5a** by ten, as a determination value. Here, if the blood plasma (blood serum) is negative with respect to red blood cells for example, then the red blood cells will precipitate at the deepest part in the center of the bottom wall **5b** of the well **5a,** without causing an agglutination reaction. For this reason, in the reaction image of the well **5a** as illustrated in Figure **12****,** the average amount of light **Lp** in a periphery part **P** of the well **5a** is comparatively greater than the average amount of light **Lc** in a center part **C** of the well **5a.** As a result, the value of the determination parameter **P/C** becomes 40 or greater. Here, Figure **12** as well as Figures **13** and **14****,** to be described below, illustrate blood cell agglutination images in a case of a reaction using a plate settling method.

On the other hand, if the blood plasma (blood serum) is a weak positive sample indicating a moderate reactivity with respect to red blood cells for example, then the red blood cells are defined to be halfway between agglutination and non-agglutination. Thus, if such weak positive blood plasma (blood serum) is processed in a similar manner, then in the reaction image of the well **5a** as illustrated in Figure **13****,** the difference between the average amount of light **Lp** in the periphery part **P** and the average amount of light **Lc** in the center part **C** of the well **5a** becomes comparatively smaller than the case of the negative sample illustrated in Figure **12****.** As a result, the value of the determination parameter **P/C** becomes within the range of 15 to 30.

If the blood plasma (blood serum) is a strongly positive sample indicating a strong reactivity with respect to red blood cells for example, then a fine agglutination mass is produced due to a reaction between the antigen present on the surfaces of red blood cells and the antibody present in the blood plasma (blood serum). Thus, if such strongly positive blood plasma (blood serum) is processed in a similar manner, then it disperses in a particle form throughout the well **5a.** Accordingly, in the case of a strongly positive sample, the average amount of light **Lp** in the periphery part **P** of the well **5a** and the average amount of light **Lc** in the center part **C** of the well **5a** are almost equal in the reaction image of the well **5a.** As a result, the value of the determination parameter **P/C** becomes within the range of 10 to 15. If the value of the determination parameter **P/C** becomes within the range of 30 to 40, then automatic determination cannot be made.

After image processing, the determination section **12a** determines the blood plasma (blood serum) to be either positive or negative for each well **5a** on the basis of the determination parameter **P/C** calculated by the image processing section **11** (step **S110).** Then, the determination section **12a** determines the blood type of the blood plasma (blood serum) on the basis of the determination result made for each well **5a** (step **S112).**

After determination of the blood type is finished, the operator opens the door **2a** of the blood cell agglutination image determining apparatus **1** upwards, and detaches the bucket **4** retaining the microplate **5** from the rotor **3.** Next, the microplate **5** is removed from the bucket **4,** and then the bucket **4** retains a new microplate **5,** in which new blood plasma (blood serum) of an object for determination is dispensed to two wells **5a,** then a type-A red blood cell suspension and a type-B red blood cell suspension are dispensed in these two wells **5a.** Similar to the case described above, the bucket **4** retaining the new microplate **5** is supported by the rotor **3** within the chamber **2b** to repeat the blood cell agglutination image determining method.

As described above, according to the present invention, the microplate **5** is rotated to separate blood cells out towards the side closer to the bottom wall **5b,** and then, the microplate **5** is inclined so that the side of the microplate **5** in the front of the rotating direction will be downwards with respect to the vertical direction to allow the reagent to react with the blood sample, and on the basis of the reaction image, the blood sample is determined to be either positive or negative for each well **5a.** For this, the present invention requires only 15 seconds for the centrifugation processing to move blood cells towards the side closer to the bottom wall **5b** of the microplate **5,** and requires only **2** minutes to incline the microplate **5** and allow the reagent to react with the blood sample. Owing to this, even if the time required for dispensing the reagent and sample, attaching and detaching the plate to and from the bucket, and the like, and additionally for the determination, is considered, the present invention is capable of processing blood samples in a shorter time compared to the conventionally performed tube tests, column agglutination technology, or plate settling methods, thereby obtaining a reproducible determination result. Further, since the present invention determines the blood sample to be either positive or negative by centrifugation processing and inclination processing of the microplate into which the blood sample and reagent are dispensed, the determination can be performed at lower cost compared to the conventionally utilized tube tests or column agglutination technology.

### (Example)

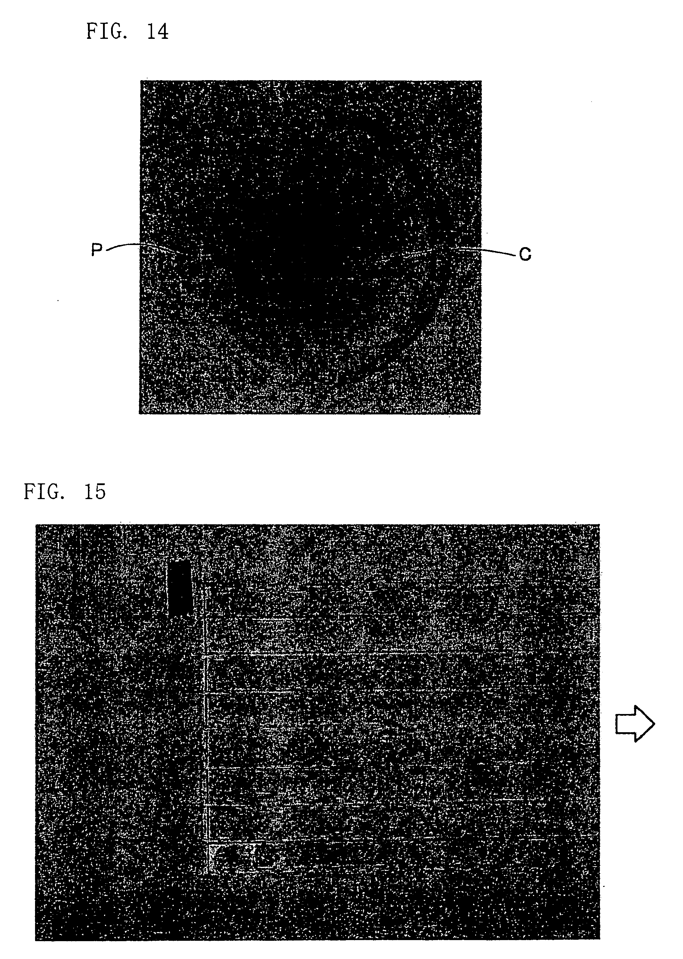

Hereinafter, an example of the present inventionwill be described. Figure **15** is a diagram of an image captured by the CCD camera **8** of a microplate **5** immediately after centrifugation processing is performed by the blood cell agglutination image determining apparatus **1.** Here, the right direction illustrated by the arrow in the figure is the rotation direction of the microplate **5.** Additionally, in the microplate **5,** 25 µL of blood plasma (blood serum) of an object for determination and 25 µL of a red blood cell suspension as a reagent are dispensed into each of a total of eight wells **5a,** the wells **5a** being in the fourth and fifth rows from the top and in the second to fifth columns from the left. Further, 0.43% of the red blood cell suspension is dispensed into the wells **5a** in the fourth row from the top, and 0.85% of the red blood cell suspension is dispensed into the wells **5a** in the fifth row.

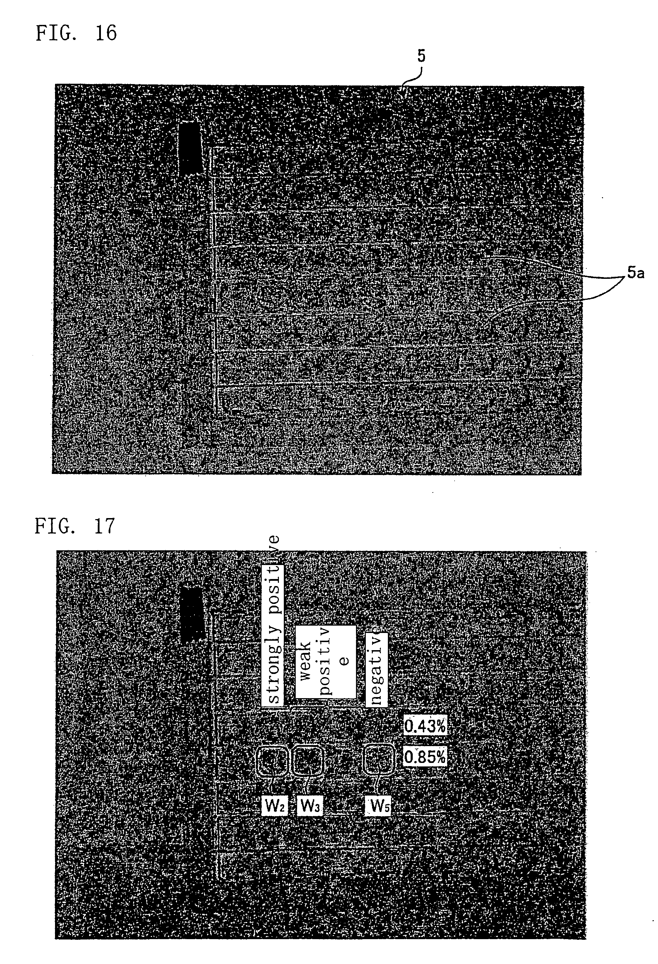

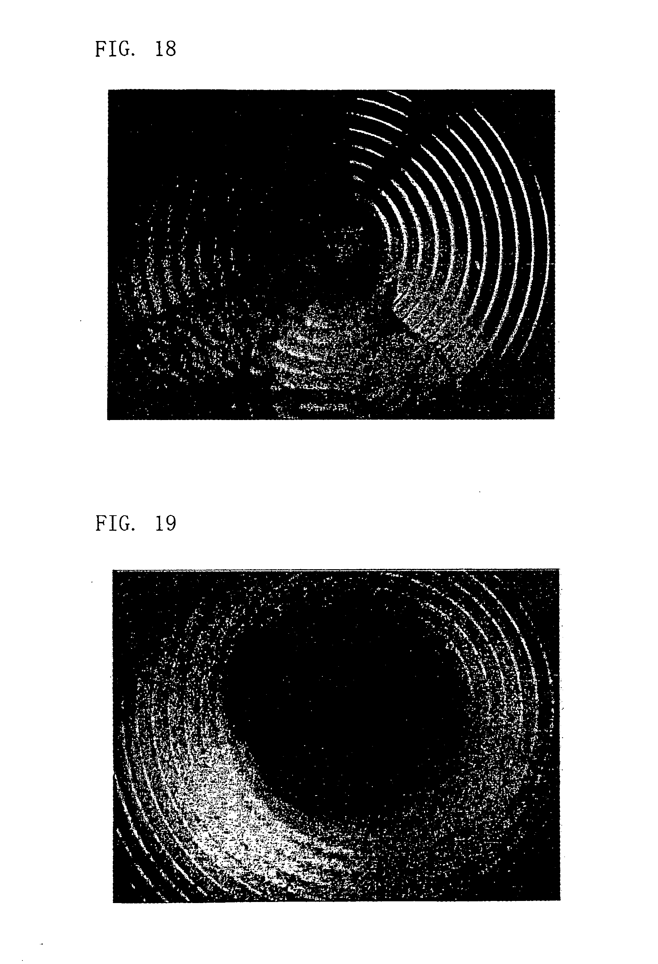

In the meantime, Figure **16** is a diagram of an image captured by the CCD camera **8** of a microplate **5** after centrifugation processing and further inclination for two minutes. Further, Figure **17** is a diagram of determination results of the images illustrated in Figure **16** together with the concentration of the red blood cell suspensions. Here, the image processing section **11** processed the image for each of the total of eight wells **5a** of the microplate **5,** including the reaction image captured by the CCD camera **8,** and the determination section **12a** determined the blood plasma (blood serum) to be either positive or negative on the basis of the obtained determination value for each well **5a.** For the determination parameter, the amount of transmitted light at the center part of the well can be applied, but it is also possible to combine it with other parameters for determination. Further, Figures **18** to **20** are diagrams of an enlarged view of the strongly positive well **5a (W2)** in the fifth row, second column; an enlarged view of the weak positive well **5a (W3)** in the fifth row, third column; and an enlarged view of the negative well **5a (W5)** in the fifth row, fifth column, among the images illustrated in Figure **17****.**

As apparent from the diagrams illustrated in Figures **18** to **20**, according to the present invention, the blood samples were able to be processed in a short time, thereby obtaining a reproducible determination result, and furthermore, the strongly positive, weak positive and negative blood samples were able to be clearly and mutually determined in a visual manner. In particular, weak positive and negative results were able to be clearly and mutually determined in a visual manner, which had been conventionally said to be difficult to determine.

### (Comparative Example)

Here, the blood cell agglutination image determining method according to the present invention and a tube test, as a comparative example of the present invention, were used to determine a blood sample three times each. In such tube testing, the intensity of agglutination is represented in such a manner as "1+" and "2+", where "2+" represents a weak positive image that can be comparatively easily determined from negative, while "1+" represents a weak positive image that requires some level of performance to determine the sample as positive (see Non-Patent Literature 1) . While the determination results of the tube test for a weak positive blood sample varied from "2+" to "1+", reaction images that were able to be readily determined to be positive were always formed using the blood cell agglutination image determining method according to the present invention, and the blood cell agglutination image determining method according to the present invention was superior to the tube test with regard to the reproducibility in the determination results of the weak positive blood sample.

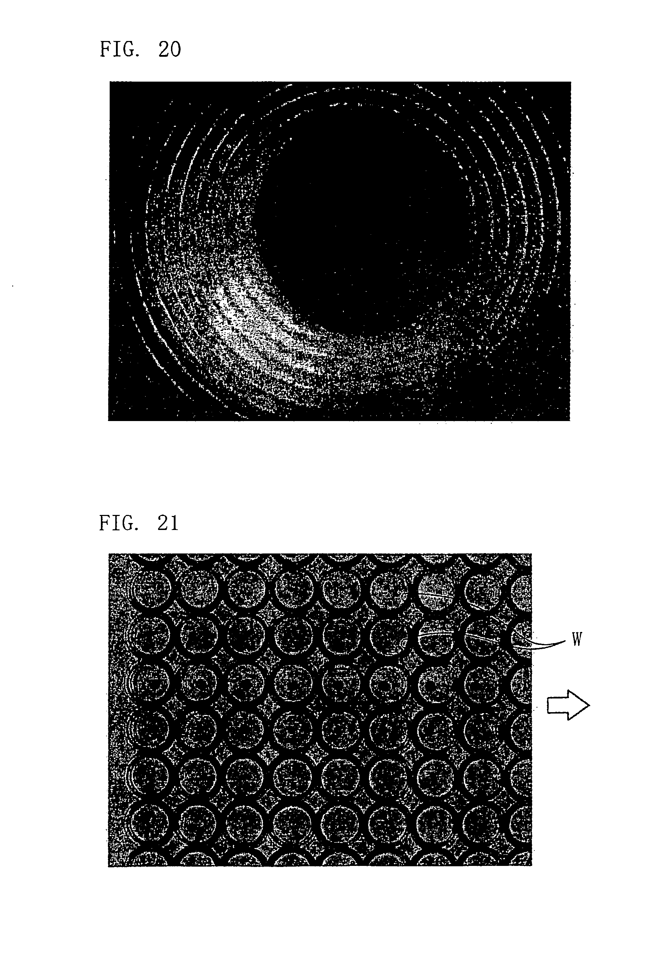

Additionally, as another comparative example of the present invention, the blood cell agglutination image determining method was performed under the same conditions as described above by the blood cell agglutination image determining apparatus **1,** using a microplate having the same number of wells **W** as the microplate 5, except that the inner surface of each bottom wall is processed to be a smooth U-shape and adj acent wells are closer to each other. The result thereof will be illustrated in Figures **21** to **25****.**

Figure **21** is a diagram of an image captured by the CCD camera **8** of the microplate immediately after centrifugation processing is performed. Here, the right direction illustrated by the arrow in the figure is the rotation direction of the microplate. In addition, in the microplate, 25 µL of blood plasma (blood serum) of an object for determination and 25 µL of a red blood cell suspension as a reagent were dispensed into each of a total of 16 wells **W** in two adjacent rows. Here, 1.7% of the red blood cell suspension was dispensed in the wells **W** on the upper row, and 0.85% of the red blood cell suspension was dispensed into the wells **W** on the lower row. Additionally, undiluted blood plasma (blood serum) was used for the four columns on the left side, and two-fold diluted blood plasma (blood serum) that was diluted with physiological saline was used for the four columns on the right side.

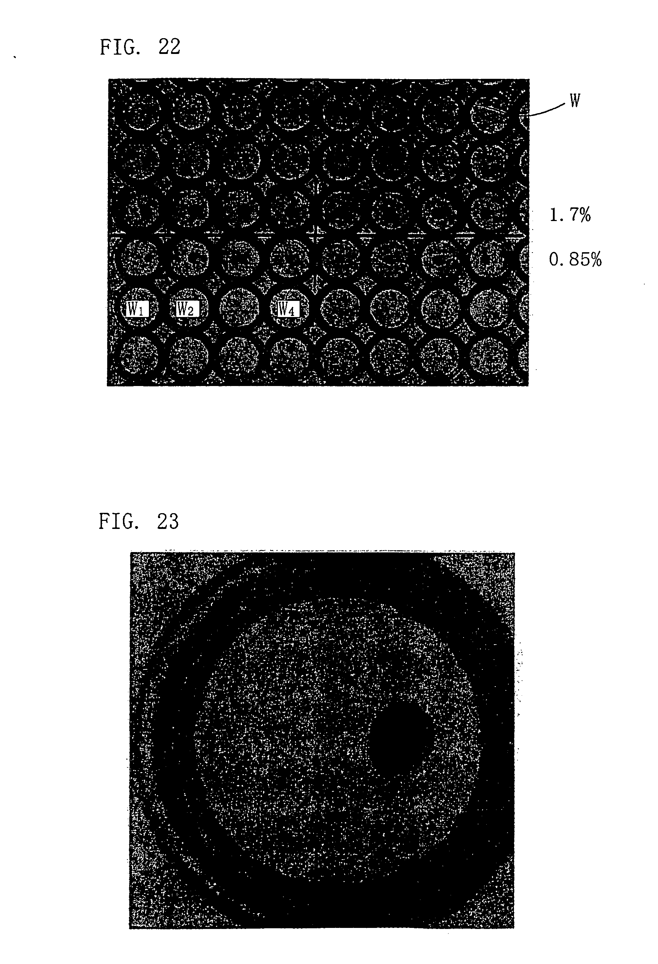

Figure **22** is a diagram of an image captured by the CCD camera **8** of the microplate after being inclined for two minutes by the inclining apparatus **7.**

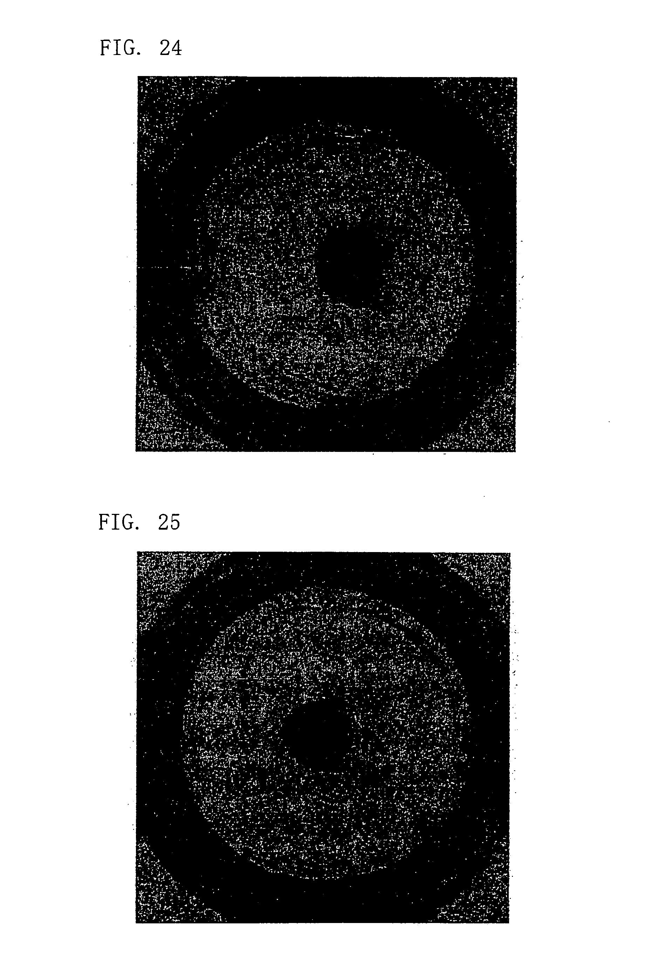

Here, Figure **23** is an enlarged view of a well **W1** in the fourth row, first column in Figure **22****,** and Figure **24** is an enlarged view of a well **W2** in the fourth row, second column thereof. In addition, Figure **25** is an enlarged view of a well **W4** in the fourth row, fourth column thereof. As apparent from Figures **23** to **25****,** in a case of the microplate having the U-shaped wells **W** each with a smooth inner surface of the bottom wall, a flow of blood cell particles is observed in the right direction from the blood cell precipitate concentrated at the well center part in the agglutination reaction image of the weak positive sample in the well **W1,** while the same degree of flow of blood cell particles is observed in the non-agglutination reaction image of the negative sample in the well **W4.** Thus, it was difficult to make a determination for the well **W2** and well **W4** based on the visual information. Accordingly, it was found desirable for the well **5a** of the microplate **5** to have the tiered portion **5c** formed on the inner surface of the bottom wall **5b.**

In the blood cell agglutination image determining method according to the present invention, it should be noted that after performing the centrifugation processing and inclination processing of the microplate **5,** the microplate **5** may be taken out of the blood cell agglutination image determining apparatus **1** to make a determination of being positive or negative by visual observation. However, making a determination in the blood cell agglutination image determining apparatus **1** provides an advantage of obtaining a determination result on the basis of a constant standard.

While the image processing section **11** performs calculation with the determination parameter **P/C** as a determination value in the embodiment described above, the embodiment is not limited to this. For example, the average amount of light **Lc** in the center part **C,** or other determination parameters may be used, and moreover, it is also possible to combine and use these determination parameters to determine if a blood sample is positive or negative.

Further, while the wells **5a** of the microplate **5** are used as reaction containers in the embodiment described above, a reaction container of an individually independent well **5a** may be used instead of the wells **5a.**

A control program for controlling the processing executed by the blood cell agglutination image determining apparatus **1** is installed on a storage section (not shown) of the control mechanism **12** illustrated in Figure **1****.** In general, installing such a control program on a memory of a computer allows the computer to function as a part or all of the control section **12** (Figure **1**). Such a control program may be installed on a memory prior to the shipping of the computer, or may be installed on a memory after the shipment of the computer. The program may be installed on a memory of the computer by reading the program recorded on a recording medium, or the program that is downloaded via a network, such as the Internet, may be installed on a memory. As to the computer, any type of computer can be used.

Once the control program is installed on a computer, the computer will function as a part or all of the control section **12** (Figure **1****).** In this case, the control section **12** (Figure **1****)** in operation means that a control method corresponding to the installed control program is being executed. This is because the control method corresponds to the operation method of the control mechanism.

As described above, the present invention is exemplified by the use of its preferred embodiment. However, the present invention should not be interpreted solely based on the embodiment described above. It is understood that the scope of the present invention should be interpreted solely based on the claims. It is also understood that those skilled in the art can implement equivalent scope of technology, based on the description of the present invention and common knowledge from the description of the detailed preferred embodiment of the present invention. Furthermore, it is understood that any patent, any patent application and any references cited in the present specification should be incorporated by reference in the present specification in the same manner as the contents are specifically described therein.

The present application claims priority to Japanese Patent Application No. 2009-293376, and it is understood that the entire contents of which are incorporated by reference herein as a part constituting the present specification in the same manner as the contents are specifically described in the present specification.

### INDUSTRIAL APPLICABILITY

As described above, the blood cell agglutination image determining apparatus and blood cell agglutination image determining method according to the present invention are useful for processing and determining a blood sample in a simple manner and in a shorter time.

### [Reference Signs List]

- **1**: blood cell agglutination image determining apparatus
- **2**: casing
- **3**: rotor
- **3d**: trunnion pin
- **4**: bucket
- **4b**: engagement groove
- **5**: microplate
- **5a**: well
- **5b**: bottom wall
- **5c**: tiered portion
- **6**: motor
- **7**: inclining apparatus
- **8**: CCD camera
- **9**: lighting
- **11**: image processing section
- **12**: control section
- **12a**: determination section
- **71**: chuck
- **72**: revolving motor
- **73**: elevating device
- **Ss**: shorter side

## Claims

1. A blood cell agglutination image determining method comprising:
a reaction step of allowing a blood sample to react with a reagent in a reaction container;
a centrifugation processing step of rotating the reaction container so that a bottom wall of the reaction container will turn outwards by centrifugal force;
an inclining step of inclining the reaction container so that a front part of the reaction container along the rotating direction in the centrifugation processing step will be downwards with respect to the vertical direction more than a back part of the reaction container; and
a determining step of determining the blood sample to be positive or negative on the basis of a blood cell agglutination image from a reaction formed in the reaction container between the blood sample and the reagent.

2. The blood cell agglutination image determining method according to claim 1, wherein the reaction container is a well formed in a microplate.

3. The blood cell agglutination image determining method according to claim 1, wherein a tiered portion is formed on an inner surface of a bottom wall of the reaction container, and the blood cell is a red blood cell.

4. The blood cell agglutination image determining method according to claim 1, wherein after the inclining step, the method further comprises:
a canceling step of canceling the inclination of the reaction container;
an image-capturing step of capturing an image of the reaction container, inclination of which is canceled; and
an image processing step of processing an image of the reaction container including a reaction image captured in the image-capturing step, and calculating a determination value on the basis of the image of the reaction container, and
wherein the determining step comprises a determining step of determining the blood sample to be positive or negative on the basis of the determination value calculated in the image processing step.

5. A blood cell agglutination image determining apparatus comprising:
a reaction container housing section for housing a reaction container for allowing a blood sample to react with a reagent therein;
rotating means for rotating the reaction container so that a bottom wall of the react ion container will turn outwards by centrifugal force;
inclining means for inclining the reaction container so that a front part of the reaction container along the rotating direction will be downwards with respect to the vertical direction more than a back part of the reaction container; and
determining means for determining the blood sample to be positive or negative on the basis of a blood cell agglutination image from a reaction formed in the reaction container between the blood sample and the reagent.

6. The blood cell agglutination image determining apparatus according to claim 5, wherein the reaction container is a well formed in a microplate.

7. The blood cell agglutination image determining apparatus according to claim 5, wherein a tiered portion is formed on an inner surface of a bottom wall of the reaction container, and the blood cell is a red blood cell.

8. The blood cell agglutination image determining apparatus according to claim 5,
wherein the rotating means comprises:
a motor; and
a rotor rotated by the motor, and
wherein the reaction container housing section comprises a bucket for retaining the reaction container, the bucket being swingably supported by the rotor around the horizontal axis.

9. The blood cell agglutination image determining apparatus according to claim 5, further comprising:
image-capturing means for capturing an image of the reaction container; and
image processing means for processing an image of the reaction container including a reaction image captured by the image-capturing means, and calculating a determination value on the basis of the image of the reaction container,
wherein the determining means comprises determining means for determining the blood sample to be positive or negative on the basis of the determination value.

10. The blood cell agglutination image determining apparatus according to claim 6, further comprising:
image-capturing means for capturing an image of a plurality of the wells; and
image processing means for processing an image of each of the wells including a reaction image captured by the image-capturing means, and calculating a determination value on the basis of the image of each of the wells,
wherein the determining means comprises determining means for determining the blood sample to be positive or negative for each of the wells on the basis of the determination value.

11. The blood cell agglutination image determining apparatus according to claim 5, wherein the inclining means comprises:
holding means for holding an outer edge of the reaction container housing section;
revolving means for revolving the holding means to incline the reaction container housing section so that a shorter side of the reaction container in the front of the rotating direction will be downwards with respect to the vertical direction; and
elevating means for elevating the revolving means together with the holding means.

12. The blood cell agglutination image determining apparatus according to claim 6,
wherein the rotating means comprises:
a motor; and
a rotor rotated by the motor, and
wherein the reaction container housing section comprises a bucket for retaining the microplate, the bucket being swingably supported by the rotor around the horizontal axis, and
wherein the inclining means comprises:
holding means for holding an outer edge of the bucket;
revolving means for revolving the holding means to incline the bucket so that a shorter side of the microplate in the front of the rotating direction will be downwards with respect to the vertical direction; and
elevating means for elevating the revolving means together with the holding means.

13. A control program for sample processing in a blood cell agglutination image determining apparatus for determining a blood sample to be positive or negative on the basis of a blood cell agglutination image of a reaction between the blood sample and a reagent in a reaction container, the control program for implementing processing that is executed by the blood cell agglutination image determining apparatus in accordance with an instruction from an operator, the processing comprising:
a centrifugation processing procedure for rotating the reaction container so that a bottom wall of the reaction container will turn outwards by centrifugal force; and
an inclining procedure for inclining the reaction container so that a front part of the reaction container along the rotating direction in the centrifugation processing step will be downwards with respect to the vertical direction more than a back part of the reaction container.

14. A computer-readable recording medium recording a control program for sample processing in a blood cell agglutination image determining apparatus for determining a blood sample to be positive or negative on the basis of a blood cell agglutination image of a reaction between the blood sample and a reagent in a reaction container, the control program for implementing processing that is executed by the blood cell agglutination image determining apparatus in accordance with an instruction from an operator, the processing comprising:
a centrifugation processing procedure for rotating the reaction container so that a bottom wall of the reaction container will turn outwards by centrifugal force; and
an inclining procedure for inclining the reaction container so that a front part of the reaction container along the rotating direction in the centrifugation processing step will be downwards with respect to the vertical direction more than a back part of the reaction container.
